# EUROPEAN PATENT APPLICATION

(11) **EP 2 311 443 A1**
(43) Date of publication of application: **20.04.2011**
(21) Application number: 09013039.4
(22) Date of filing: 15.10.2009
(51) Int. Cl.: A61K 31/00, A61K 31/357, A61K 31/415, A61K 31/4706, A61K 31/49, A61K 45/06, A61P 33/00, A61P 33/02, A61P 33/08

(54) **Pharmaceutical composition containing cannabinoid-receptor 2 antagonists**

(71) Applicant: Rheinische Friedrich-Wilhelms-Universität, 53113 Bonn (DE)
(72) Inventor: Alferink, Judith, 53111 Bonn (DE); Zimmer, Andreas, 53125 Bonn (DE); Hörauf, Achim, 53117 Bonn (DE); Specht, Sabine, 53111 Bonn (DE)
(74) Representative: Kröncke, Rolf

(57) **Abstract**

The present invention relates to the use of cannabinoid-receptor 2 (CB₂R)-antagonists for the prophylaxis and/or treatment of pathogen, in particular, parasitic protozoa, caused or induced cerebral diseases, disorders or conditions including secondary and accompanying diseases, disorders or conditions, like encephalitis. In a preferred embodiment, said CB₂R-antagonist is a 3-pyrazole-carboxamide derivative. In a further aspect, the present invention provides pharmaceutical compositions comprising said CB₂R-antagonist in combination with an anti-pathogenic active ingredient, optionally together with pharmaceutically acceptable carriers, diluents and/or excipients. Said pharmaceutical compositions are useful for treating encephalitis, like cerebral malaria induced by *Plasmodium falciparum.* Thus, in another aspect, methods for the treatment of pathogen, like parasitic protozoa induced or caused cerebral diseases, disorders or conditions including encephalitis, in particular cerebral malaria are provided comprising the steps of administering a CB₂R-receptor antagonist.

## Description

The present invention relates to the use of cannabinoid-receptor 2 (CB₂R)-antagonists for the prophylaxis and/or treatment of pathogen, in particular, parasitic protozoa, caused or induced cerebral diseases, disorders or conditions including secondary and accompanying diseases, disorders or conditions, like encephalitis. In a preferred embodiment, said CB₂R-antagonist is a 3-pyrazole-carboxamide derivative. In a further aspect, the present invention provides pharmaceutical compositions comprising said CB₂R-antagonist in combination with an anti-pathogenic active ingredient, optionally together with pharmaceutically acceptable carriers, diluents and/or excipients. Said pharmaceutical compositions are useful for treating encephalitis, like cerebral malaria induced by *Plasmodium falciparum.* Thus, in another aspect, methods for the treatment of pathogen, like parasitic protozoa, induced or caused cerebral diseases, disorders or conditions including encephalitis, in particular, cerebral malaria, are provided comprising the steps of administering a CB₂R-receptor antagonist.

### Prior art

The peripheral cannabinoid receptor (CB₂R) was the second cannabinoid-receptor discovered in 1993 while the first CBR, namely, the central cannabinoid-receptor (CB₁R) was described in 1990. Both cannabinoid-receptors are G-protein-coupled seven transmembrane receptors sharing high homology between human and mouse.

The CB₁R is expressed at high levels in brain tissue and to a lesser extent in peripheral tissues, like glands, reproductive organs or immune cells. In contrast, CB₂R is present and expressed mainly in cells of haematopoietic origin. In particular CB₂R expression has been demonstrated in various organs and on various cells involved in the immune system, like spleen, thymus, lymph nodes as well as on B cells, natural killer cells and to a lesser extend on T cells. Furthermore, it was reported that the expression level of the CB₂R depends on the stage and activity of the respective cell. Moreover, recently, CB₂R expression was described in other types of cells, e.g. cells involved in the bone formation like osteoclasts and osteoblasts as well as in the central nervous system, e.g. microglial cells and astrocytes.

To allow further investigation of the role of said CBR receptors in the development and metabolism of organisms, the knockout mouse model was used knocking out either CB₁R or CB₂R. Various changes in metabolism and changes in occurrence of various types of cells can be observed. Recently, Buckley, N.E., British Journal of Pharmacology (2008), 153 309-318, reviewed the phenotypes observed in CB₁R and CB₂R knockout mice and its use in investigating various disease models.

To summarize, implication of the CB₂R has been reported for various diseases, including liver disorders, atherosclerosis, allergic dermatitis, pain, multiple sclerosis, osteoporosis etc. As a model for multiple sclerosis, experimental autoimmune encephalomyelitis was investigated in the CB₂R knockout mouse. Further, said mouse model was used as infectious model for determining immune responses to various bacterial derived diseases. In this connection, known CB₁R- and CB₂R-antagonists were used to determine effects of antagonists in said models and it is described that antagonists only partial attenuated e.g. THC induced suppression of IL-12 production by cells obtained from knockout mice. Furthermore, this review reports on examination of knockout mice infected with *Plasmodium berghei* ANKA. Infection of wild type mice with *Plasmodium berghei* ANKA is a model for cerebral malaria. Typically, said infection leads to 100 % lethality after 6 to 7 days of infection. It was described that the CB₂R knockout mice are resistant to cerebral disease after being infected with this parasite. The reason for the resistance of said knockout mice to cerebral malaria is not known until today.

As mentioned before, antagonists of both CBRs are known in the art. For example the compound SR144528 represents a known CB₂R-antagonist while SR141716A is a known CB₁R-antagonist. As identified in the review of Buckley (supra) said antagonist may have various effects in CB₂R mouse models and the use of said antagonists, like the molecule SR144528 is noted for various types of diseases, like allergic dermatitis, hypertension, atherosclerosis etc.

A well-known CB₂R-antagonist is SR144528 of Sanofi-Aventis. Said molecule was first described in WO 97/21682. Therein, the use of 3-pyrazole-carboxamide derivatives having cannabinoid-receptor affinity is disclosed. Diseases, disorders or conditions where said derivatives should be useful include immune disorders, like autoimmune diseases, allergic diseases as well as infectious diseases.

Investigations into the function and involvement of genes in the physiology and development of organisms are often performed with so called knockout mice. In knockout mice specific predetermined genes are switched off by homologous recombination in embryonic stem cells. However, it should be noted that in the most common implementation of the knockout technology, the constitutive invalidation of genes, affects the entire organism, starting from the embryonic development to the generation of organs, cells etc. This technology is therefore often not suitable to draw any conclusions about specific metabolic or physiological functions of the said gene or gene products.

Another approach to examine the function of gene products with molecules influencing specifically the gene product is the pharmacological approach. For example, wild type mice are treated with molecules altering the expression or properties of the molecule to be investigated. A common implementation of this methodology involves molecules acting as agonist, antagonist, inverse agonists etc., which relates to the functional changes of the target structure caused by allosteric or competitive interaction with such molecules. Another implementation of the technology involves molecules that affect the synthesis and metabolism of the target gene product and may thus result in a silencing of said proteins or genes encoding for said proteins. There are numerous examples showing that comparing the results obtained from knockout technology with results obtained by "traditional" pharmacological approaches, the results obtained from knockout mice can not allow deduction to pharmacology. For example, in various knockout mice, unknown gene function with respect to organ development or cellular composition in the organism are made responsible for additional effects, which can not be verified when applying a pharmacological approach, see e.g. Wang, J., J Clin Invest. 2005 115(3):711-7, Vandenbroeck K. et al., J. Pharm Pharmacol, 2004;56(2): 145-60). For example Wang, supra, demonstrates that HVEM (herpesvirus entry mediator) knockout T-cells display an increased response upon *in vitro* stimulation with Concanavalin A compared to wild type T-cells. In contrast, pharmacological approaches revealed that HVEM exerts costimulatory functions in the immune response mediated by T cells. Further, it was postulated that endogenous IL-12 is not involved in insulin dependent diabetes mellitus (IDDM) since IL-12 deficient mice develop insulitis similar to wild type mice. In contrast, pharmacological studies, namely, treating animals with IL-12 antagonists, demonstrate that a protection against IDDM can be achieved (Trembleau S, J of Immunology, 1999, 163: 2960-2968.; Rothe H, et al., Diabetologia. 1997 Jun;40: 641-6).

In particular in the field of the endogenous cannabinoid system, various examples are known demonstrating that it is not possible to draw any conclusions on the effect of modulators, like agonist or antagonist of the cannabinoid-receptors, based on the phenotypes observed in knockout mice. For example, CB₂-receptor deficient mice demonstrate a significantly enhanced contact allergy. In contrast, the known CB₂-receptor agonist HU308 does not show an effect on this disease (Karsak et al., Science, 2007,8;316:1494-7). The endocannabinoid anandamide displays analgesic effect in animals which do not express either CB₁ or CB₂ receptors (Racz et al., European Journal of Pharmacology, 2008, 596:98-101). CB₁ receptor antagonists are able to block the uptake of milk of new born animals. In contrast, CB₁ knockout mice do not show any abnormalities in this regard.

In CB₂-receptor deficient animals, there can be observed a significant change in the structure of the spleen, a reduced number of B-cells, CD4 memory T-cells, and NK T-cells (Ziring et al., Immunogenetics, 2006, 58(9):714-25). Said changes in structure and number of cells are the result of CB₂-receptor deficiency during embryonic development of the animal. It can not be expected that said changes can be achieved when applying pharmacologicals.

In contrast to the knockout technology, which results in a permanent disruption of the specific gene and, consequently, a permanent silencing of said gene started from the beginning of the embryonic development; pharmacological inhibition results in a transient alteration of the target molecule only. Changes observed in knockout mice, typically, can not be reflected by transient administration of pharmacologicals. Consequently, it is not possible to transfer data obtained from the knockout technology on pharmacological approaches as identified above.

Encephalitis as a form of cerebral diseases, disorders or conditions, is an acute inflammation of the brain. Pathogen infections may induce or cause encephalitis. In the literature, it is differentiated between forms of encephalitis wherein the pathogen cause the inflammation of the brain directly, e.g. bacterial meningitis or viral encephalitis, sometimes called primary encephalitis in the literature. On the other hand, encephalitis may be a secondary or accompanying disease, e.g. a complication of a current infectious disease induced by the pathogen. Typically this type of encephalitis occurs in parasitic infections, such as toxoplasmosis, malaria, or primary amoebic meningoencephalitis, sometimes called secondary encephalitis in the literature. Further, Lyme disease may cause encephalitis as Bartonella henselae can do. Moreover, encephalitis can be observed after viral or bacterial infections, like infection with Borrelia burgdorferi, treponema pallidum, Listeria monocytogenes: Herpes varizella, Varizella-Zoster-Virus, Coxsackie- and Enterovirus, Epstein-Barr-Virus, mumps-Virus, measles-Virus, Herpes simplex type 2, LCM-Virus, HIV, etc.

Malaria as a typical example of parasitic protozoa induced diseases has a tremendous impact on human health, killing millions annually, and the disease is a major impediment for social and economic development of nations in malaria-endemic areas, in particular, in sub-Saharan Africa. Infection of protozoa of the genus Plasmodium typically develops to the disease identified as malaria. Various forms of malaria exist, for example, malaria tropica is induced by infection with *Plasmodium falciparum* while malaria tertiana is induced by *Plasmodium vivax. Plasmodium falciparum* and *Plasmodium vivax* are the most common types of Plasmodium. Typically, the group of human pathogenic *Plasmodium* species is referred to as malaria parasites.

Cerebral malaria (CM) involves the clinical manifestations of *Plasmodium falciparum* malaria further inducing changes in mental status and coma. It is an acute, wide-spread disease of a brain which is accompanied by fever. The mortality range is between 25 to 50 %. If a person is not treated, CM is fatal in 24 to 72 hours. Clinical manifestations of cerebral malaria are numerous, but there are some primary symptoms generally common both to adults and children:
1. impaired consciousness with non specific fever;
2. generalized convulsions and neurological sequelae; and
3. coma that persists for 24 to 72 hours, initially rousable and than unrousable.

Cerebral malaria represents one of the known forms of severe malaria when *P.falciparum* infections are complicated by serious organ failure or abnormalities in the patients' blood or metabolism. Cerebral malaria is one form of an acute inflammation of the brain, also known as encephalitis. As mentioned before, other types of encephalitis caused by certain parasitic or protozoae infestations, such as toxoplasmosis or primary amoebic meningoencephalitis are known.

As e.g. cerebral malaria as a form of encephalitis and as a form of a disease induced by parasitic protozoa is fatal within days of infection if left untreated, immediate treatment is crucial. Treatment of malaria as a vector-borne infectious disease caused by protozonal parasites is treated by the use of plasmodium specific drugs, thus, preventing expansion of the parasites and further infection of cells in the host. Today, there are several families of drugs used to treat malaria, such as chloroquine, chinine and artemisinin. Chloroquine and chinine represent cheap and very effective drugs. It therefore represented the anti-malaria drug of choice for many years in most parts of the world. Artemisinin-based therapies should have improved antiparasitic treatment, but in-hospital mortality still remains high, as do neurological sequelae. The excessive activation of the immune system plays an important role in the pathogenesis of the disease. Drugs capable of modulating the immune response in addition to drugs with antiplasmodial activity have been evaluated. Antibodies to TNF-alpha, pentoxifylline, and thalidomide have been tried for this purpose with variable success.

Thus, there is still an emerging need of new drugs or combination of drugs for fighting against pathogen induced infections, like encephalitis, like parasitic infection, in particular, malaria and secondary and accompanying diseases, disorders or conditions, in particular, cerebral malaria. Also other parasitic protozoa inducing strong inflammation and thereby pathology, like toxoplasmosis, should be considered. Toxoplasmosis is induced by *Toxoplasma gondii* protozoa of the class of *Coccidia.* Other examples of protozoa as members of the coccidiae belonging to the class *Apicomplexa* are the genus of *Eimeria, Isospora, Sarcocystis, Hammonida, Neospora* and *Cryptosporidium.* Both, *Plasmodia* and *Coccidiae* are members of the class of *Apicomplexa.* Another important class of parasitic protozoa inducing infectious diseases in man and animal is *Flagellata. Trypanosoma cruzi,* the cause of chagas disease and *Trypanosoma brucei,* the cause of sleep sickness as well as *Leishmania,* the cause of leishmaniosis, are examples of *Flagellata.* For the above mentioned diseases, cerebral diseases, disorders or conditions, in particular, encephalitis, may be involved in the clinical picture of the pathogen induced infection.

In view of the above, it is clear that there is an urgent need for new drugs preventing or combating symptoms, disorders or conditions induced by or caused by pathogens like parasitic infections, preferably, of pathogen induced encephalitis. In particular, there is an urgent need for the development of drugs to treat the cerebral or neurological symptoms, like encephalitis, of pathogen infection, like parasitic protozoa infection, which have a rapid onset and a high mortality.

Thus, the object of the present invention is to provide new substances to fight against cerebral diseases, disorders or conditions induced by or caused by pathogens in general, like parasitic protozoa, in particular, *Apicomplexa* and *Flagellata.* Particularly preferred, said new compounds are able to treat or prevent diseases, disorders or conditions caused by or induced by plasmodia, in particular preferred, to prevent or treat cerebral malaria or other types of encephalitis.

### Summary of the invention

The present invention provides pharmaceutical compositions comprising cannabinoid-receptor 2 (CB₂R)-antagonist for the use in the prophylaxis and/or treatment of pathogen, e.g. parasitic protozoa, caused or induced cerebral diseases, disorder or conditions including secondary and accompanying diseases, disorder or conditions, in particular, encephalitis.

The present inventors recognized that said CB₂R-antagonists are inter alia able to prevent *Plasmodium berghei* ANKA induced death in animals. Said animals otherwise die due to cerebral malaria if not treated with a CB₂R-antagonist.

In a further aspect, the present invention relates to a pharmaceutical composition comprising a CB₂R-antagonist and at least one further anti-pathogenic active ingredient, e.g. an anti-protozoal active ingredient, optionally, together with pharmaceutically acceptable carriers, diluents and/or excipients.

In a preferred embodiment, said pharmaceutical composition is a combination of a CB₂R-antagonist and chloroquine or chinines. or artemisinins.

In a preferred embodiment, said CB₂R-antagonist is a compound according to formula I with the definitions of the substituents as provided herein. In a particular preferred embodiment, said CB₂R-antagonist is SR144528, (1S-endo)-5-(4-Chloro-3-methylphenyl)-1-((4-methylphenyl)methyl)-N-(1,3,3-trimethylbicyclo(2.2.1)hept-2-yl)-1H-pyrazole-3-carboxamide).

### Brief descriptions of the drawings

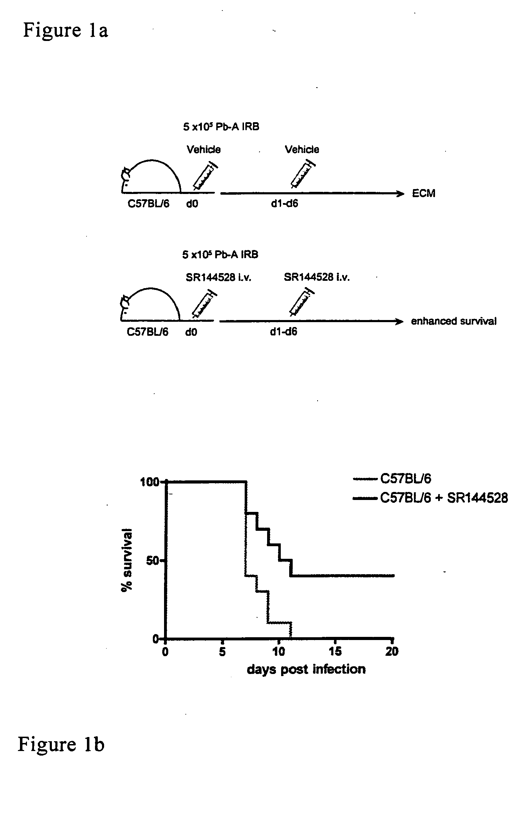

- Figure 1:: In figure 1, the experimental design, figure la, and the survival rate after infection, 1 b, is shown.

### Detailed descriptions of the invention

The present inventors recognized that CB₂R-receptor antagonists are able to prevent or treat pathogen, e.g. parasitic protozoa, induced or caused cerebral diseases, disorders or conditions, in particular pathogen induced encephalitis.

That is, it was surprisingly found that CB₂R-antagonists display beneficial effects in pathogen, e.g. parasitic protozoa, induced cerebral diseases, disorders or conditions in animals including humans.

Said parasitic protozoa caused or induced cerebral diseases include secondary and accompanying diseases, disorders or conditions. That is, said CB₂R-antagonists are useful for the prophylaxis and/or treatment of diseases, disorders or conditions induced by infection with protozoa, in particular, *Flagellata* like *Leishmania, Trypanosoma, Lamblia, Trichomonas* or *Apicomplexa,* like *Plasmodia* or *Toxoplasma, Eimeria, Babesia, Isospora, Theileria, Neospora, Cryptosporidia,* etc. as well as secondary and accompanying diseases, disorders or conditions present in a man or animal, like mammals, resulting from infection with said parasitic protozoa. Said secondary diseases, disorders or conditions are in particular encephalitis, like cerebral malaria occurring in severe types of malaria after *Plasmodium falciparum* infection.

In a preferrred embodiment, the present invention relates to pharmaceutical compositions comprising the CB₂R-antagonists for the prophylaxis and treatment of pathogen induced encephalitis. Said types of encephalitis include bacterial induced encephalitis as well as virus induced encephalitis. For example, the encephalitis may be primary induced by virus, like Arbovirus (FSME), HSV, Varizella-Zoster-virus, Coxsackie- and enterovirus, Epstein-Barr-virus, HIV, measles, rabies-virus, Herpes simples type 2, LCM-virus, or mumps virus.

On the other hand, the encephalitis may be a form of encephalitis due to complications of viral or bacterial infections as well as complications of parasitic protozoa infection. For example, for diseases, disorders or conditions caused and induced by infection with Borrelia burgdorferi, treponema pallidum, Listeria monocytogenes: Herpes , varizella, Varizella-Zoster-Virus, Coxsackie- und Enteroviren, Epstein-Barr-Virus, Mumps-Virus, Masern-Virus, Herpes simplex Typ 2, LCM-Virus., HIV, etc involvement of encephalitis may occur. The same holds true for infections with parasitic protozoa, like the parasitic protozoa described herein

The antagonist may be present as the free base or free acid or may be present in various types of salts. Typically, said active ingredients are provided e.g. in form of hydrates or solvates of the active ingredients, or in form of their salts. In this connection, the term "pharmaceutically acceptable salts", as used herein, refers to salts which do not display any toxicological effects when administered to animals including humans. These organic or inorganic salts may be selected from the group including hydrochlorides, hydrobromides, hydroiodides, sulphates, bisulphates, nitrates, citrates, tatrates, bitatrates, phosphates, hydrogenphosphates, dihydrogenphosphates, carbonates, hydrogencarbonates, malates, maleates, fumarates, succinates, acetates, therphtalates, laureates, palmitates, and pectinates.

In a preferred embodiment, the pharmaceutical composition comprises the cannabinoid-receptor 2 antagonist in combination with an anti-pathogenic active ingredient, like an anti-protozoal active ingredient, optionally together with pharmaceutically receptor carriers, diluents and/or recipients.

According to the present invention, any route of administration may be used for administering the pharmaceutical composition according to the present invention. The pharmaceutical composition may be provided in liquid or solid form for enteral or parenteral application. Suitable administration forms include tablets, capsules, dragees, solutions, suspensions, granules, powder, like lyophylized powder. Parenteral administration may be in form of injection (intramuscular, subcutaneous, intraveneous, intraperitoneal, intraocular); or by controlled-release forms. The route of administration further includes topical or dermal administration or administration by inhalation, like gels, emulsions or suspensions, solid preparations like powder, premix or concentrates, granules, pallets, tablets, capsules, aerosols, inhalants, etc.

In a preferred embodiment, the pharmaceutical composition is suitable for the prophylaxis and/or treatment of a cerebral disease caused or induced by protozoa belonging to *Apicomplexa* or *flagellata.* In particular, said protozoa belonging to the class of *Plasmodium, Babesia, Theileria, Toxoplasma, Eimeria, Isospora, Sarcocystis, Hammonida, Neospora, Cryptosporidium, Leishmania, Trypanosoma, Lamblia, Trichomonas.*

In a further preferred embodiment, the pharmaceutical composition is suitable for the prophylaxis and/or treatment of encephalitis caused or induced by a pathogen, like bacteria, virus or parasites, in particular, induced by parasitic protozoa.

Said pharmaceutical composition can be used for the treatment of encephalitis caused or induced by pathogens, in particular of malaria, toxoplasmosis, leishmaniosis, periplasmosis, coccidiosis, chagas disease and sleeping sickness as well as accompanying diseases, disorders or conditions in particular, cerebral malaria.

The pharmaceutical composition may optionally contain pharmaceutically acceptable carrier, diluents or excipients. In addition, additional auxiliary compounds may be present in the pharmaceutical compositions, like preservatives, antioxidants, chelating agents, binding agents etc. As mentioned before, in an embodiment of the present invention, the pharmaceutical composition contains at least one CB₂R-antagonist and at least one anti-pathogenic acitive ingredient, like an anti-protozoal active ingredient. Preferably, said anti-protozoal active ingredient is selected from the group of fluorochinolons, artemether, chloroquine, proguanil, mefloquine, lumefantrine, chinine, doxycycline, halofantrine, primaquine, sulfadoxine, tetracycline, pyrimethamine, atovaquone, cycloguanil, and/or artemisinine.

The exact dosage is chosen by the individual physician in view of the individual to be treated. Dosage and administration are adjusted to provide sufficient levels of the active moiety or to maintain the desired effect. Additional factors that may be taken into account include age and weight of the individual, diet, time and frequency, the way of administration, drug combination(s), reaction sensitivity and tolerance/response to therapy.

The pharmaceutical composition according to the present invention is preferably adapted for administration by the oral, parenteral, topical or dermal route or by inhalation.

The use of the combination of the CB₂R-antagonist and the at least one anti-protozoal active ingredient allows to enhance the anti-parasitic efficacy and the beneficial effects on possibly accompanying diseases, disorders or conditions of the compounds.

The at least two active ingredients present in the pharmaceutical composition according to the present invention as defined herein may be provided in a form to allow simultaneous, separate or sequential administration of the active ingredients. In case of solids, the active ingredients may be prepared to solid pharmaceuticals, e.g. pressed in tablets.

In a preferred embodiment, the pharmaceutical composition according to the present invention comprises a CB₂R-antagonist which is a compound according to formula I 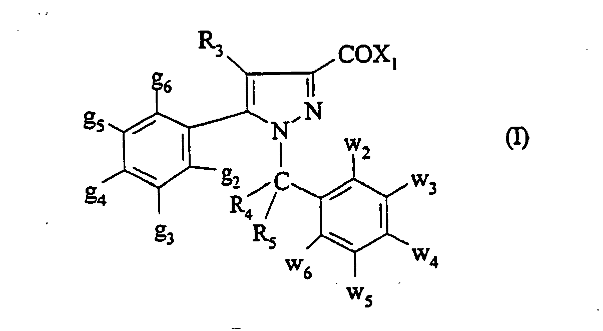 wherein X₁ is a group -NR₁R₂ or a group -OR₂;
g₂, g₃, g₄, g₅, g₆, and w₂, w₃, w₄, w₅, w₆ are identical or different and are each independently hydrogen, a halogen atom, a (C₁-C₄)alkyl, a (C₁-C₄) alkoxy, a trifluoromethyl, a nitro or a (C₁-C₄) alkylthio, with the proviso that a least one of the substituents g₂, g₃, g₄, g₅, g₆ and at least one of the substituents w₂, w₃, w₄, w₅, w₆ are other than hydrogen;
R₁ is hydrogen or a (C₁-C₄) alkyl;
R₂ is a non-aromatic (C₃-C₁₅) carbocyclic radical which is unsubstituted or monosubstituted or polysubstituted by a substituent selected from a halogen atom, a (C₁-C₄) alkyl and a (C₁-C₄) alkoxy;
-R₃ is hydrogen or a group -CH₂R₆;
R₄ and R₅ are each independently a hydrogen, a (C₁-C₄) alkyl or a trifluoromethyl;
- or else R₄ is hydrogen and R₅ and w₆ together constitute an ethylene or trimethylene radical; and
R₆ is hydrogen, or when the substituents g₂, g₃, g₄, g₅ and/or g₆ are other than a (C₁-C₄), R₆ is hydrogen, a (C₁-C₄) alkyl, a fluorine, a hydroxyl, a (C₁-C₅) alkoxy, a (C₁-C₅) alkylthio, a hydroxyl (C₁-C₅) alkoxy, a cyano, a (C₁-C₅) alkylsulfinyl or a (C₁-C₅) alkylsulfonyl; and its salts.

Preferably, said compound according to formula I is a compound with X₁ is a group -NR₁R₂ in which R₁ is hydrogen and R₂ is 1,3,3-thrimethylbicyclo [2.2.1]hept-2-yl radical or a bicycle[3.2.1]oct-3-yl radical, and its salts.

In a particular preferred embodiment, the substituents g₄ is a halogen, w₄ is a (C₁-C₆) alkyl group, R₁, R₃, R₄, R₅ are hydrogen.

Particular preferred, the CB₂R-antagonist is SR144528 (1S-endo)-5-(4-Chloro-3-methylphenyl)-1-((4-methylphenyl)methyl)-N-(1,3,3-trimethylbicyclo(2.2.1)hept-2-yl)-1H-pyrazole-3-carboxamide).

The present inventors identified that the CB₂R-antagonist allows to prevent and/or treat cerebral diseases, disorders or conditions, in particular, encephalitis, like cerebral malaria, in an individual.

In a further preferred embodiment, the CB₂R-antagonist, e.g. the CB₂R-antagonist according to formula I is used in combination with at least one of chloroquine, chinine or artemisinin.

The pharmaceutical composition of the present invention may further comprise a pharmaceutical acceptable carrier, excipients and/or diluent. Examples of suitable pharmaceutical carriers are well known in the art and include phosphate buffered solutions, sterile solutions, etc. Compositions comprising such carriers can be formulated by well known conventional methods.

In a further embodiment, the present invention relates to a method for preventing or treating parasitic protozoa caused or induced cerebral diseases, disorders or conditions including secondary and accompanying diseases, disorders or conditions, comprising the step of administering a cannabinoid-receptor 2 antagonist to an individual in need thereof. The CB₂R-antagonist is administered in an effective amount for prophylactic or therapeutic treatment. In this connection, the term "effective amount" refers to an amount or dosage of a composition sufficient to induce the desired response, in particular, to induce prophylactic therapeutic treatment of parasitic protozoa induced or caused cerebral diseases, disorder or conditions. Preferably, the method allows the treatment of cerebral malaria or other types of encephalitis.

That is, the present invention relates to a method for preventing or treating pathogen induced or caused encephalitis, either primary or secondary encephalitis comprising the step of administering a cannabinoid-receptor 2 antagonist to an individual in need thereof. The CB₂R-antagonist is administered in an effective amount for prophylactic or therapeutic treatment. Said encephalitis may be caused or induced by viral infections, bacterial or parasite infection.

The following examples illustrate representative embodiments now contemplated for practizising the invention, but should not be construed to limit the invention.

### Examples

To determine the effect of the CB2R antagonist SR144528SR in a mouse model of cerebral malaria, C57BL/6 mice were infected with *Plasmodium berghei* ANKA and intraperitoneally treated with the CB2R antagonist SR144528 or vehicle control (25 µg/mouse) on day 0 and every day thereafter, see figure 1a. Vehicle control treated C57BL/6 mice developed murine CM between day 7 and 10 after infection, and their mortality was 100% (see figure 1b). In contrast, SR144528 treated C57BL/6 mice were markedly protected from cerebral malaria, with a mortality of only 40% at day 9 (see figure 1b).

### Material and Methods

12 week old female C57BL/6 mice (n=12) were i.p injected. with 5 × 10⁴ *P. berghei* ANKA-infected red blood cells. CB2R antagonist SR144528 or vehicle control was injected intraperitoneally (25 µg/mouse). Experimental mice were monitored and scored daily for signs of severe CM (ataxia, convulsion, coma) and survival was assessed. Tail blood smears were taken and stained with Giemsa for verification of parasitemia. Mice which were resistant to CM developed anemia caused by rising parasitemia and were killed on day 22.

## Claims

1. Pharmaceutical composition comprising a CB₂R-antagonist and, optionally, pharmaceutically acceptable diluents, carrier or excipients for the use in the prophylaxis and/or treatment of pathogen induced cerebral diseases, disorders or conditions.

2. Pharmaceutical composition according to claim 1 wherein the cerebral disease, disorder or condition is an encephalitis, in particular, parasitic protozoa induced or caused encephalitis.

3. Pharmaceutical composition according to claim 1 or 2 wherein the parasitic protozoa is selected from the group of *Apicomplexa* or *Flagellata.*

4. The pharmaceutical composition according to any one of claims 1 to 3 for the use in prophylaxis and/or treatment of *plasmodium, babesia, theileria, toxoplasma, eimeria, isospora, sarcocystis, hammonida, neospora, cryptosporidium, leishmania, trypanosoma, lamblia, trichomonas* induced cerebral disease, disorder or condition, in particular, encephalitis.

5. The pharmaceutical composition according to any one of the preceding claims for use in prophylaxis and/or treatment of cerebral forms of malaria, toxoplasmosis, leishmaniosis, periplasmosis, coccidiosis, chagas disease and sleeping sickness, in particular, cerebral malaria.

6. The pharmaceutical composition according to any one of the preceding claims whereby said composition is adapted for a route of administration selected from oral, parenteral, topical, or dermal administration or administration by inhalation.

7. A pharmaceutical composition comprising a CB₂R-antagonist and at least one further anti-pathogenic active ingredient, preferably an anti-protozoal acitive ingredient, optionally together with pharmaceutically acceptable carriers, diluent and/or excipients.

8. The pharmaceutical composition according to claim 7 wherein the at least one further anti-protozoal active ingredient is selected from the group of fluorochinolons, artemether, chloroquine, proguanil, mefloquine, lumefantrine, chinine, doxycycline, halofantrine, primaquine, sulfadoxine, tetracycline, pyrimethamine, atovaquone, cycloguanil, and/or artemisinine.

9. The pharmaceutical composition according to any one of claims 7 to 8 for use in the prophylaxis and/or treatment of any one of the diseases identified in claims 1 to 5.

10. The pharmaceutical composition according to any one of claims 7 to 9 whereby the at least two active ingredients present in said pharmaceutical composition are provided in a form for simultaneous, separated or sequential administration.

11. The pharmaceutical composition according to any one of the preceding claims wherein the CB₂R-antagonist is a compound according to formula 1 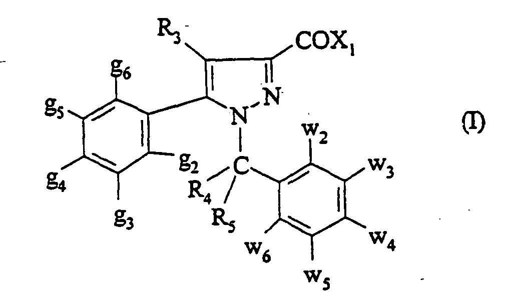 wherein X₁ is a group -NR₁R₂ or a group -OR₂;
g₂, g₃, g₄, g₅, g_{6,} and w₂, w₃, w₄, w₅, w₆ are identical or different and are each independently hydrogen, a halogen atom, a (C₁-C₄)alkyl, a (C₁-C₄) alkoxy, a trifluoromethyl, a nitro or a (C₁-C₄) alkylthio, with the proviso that a least one of the substituents g₂, g₃, g₄, g₅, g₆ and at least one of the substituents w₂, w₃, w₄, w₅, w₆ are other than hydrogen;
R₁ is hydrogen or a (C₁-C₄) alkyl;
R₂ is a non-aromatic (C₃-C₁₅) carbocyclic radical which is unsubstituted or monosubstituted or polysubstituted by a substituent selected from a halogen atom, a (C₁-_{C4}) alkyl and a (C₁-C₄) alkoxy;
-R₃ is hydrogen or a group -CH₂R₆;
R₄ and R₅ are each independently a hydrogen, a (C₁-C₄) alkyl or a trifluoromethyl;
- or else R₄ is hydrogen and R₅ and w₆ together constitute an ethylene or trimethylene radical; and
R₆ is hydrogen, or when the substituents g₂, g₃, g₄, g₅ and/or g₆ are other than a (C₁-C₄), R₆ is hydrogen, a (C₁-C₄) alkyl, a fluorine, a hydroxyl, a (C₁-C₅) alkoxy, a (C₁-C₅) alkylthio, a hydroxyl (C₁-C₅) alkoxy, a cyano, a (C₁-C₅) alkylsulfinyl or a (C₁-C₅) alkylsulfonyl; and its salts.

12. The pharmaceutical composition according to claim 11, wherein the compound of formula I with X₁ is a group -NR₁R₂ in which R₁ is hydrogen and R₂ is 1,3,3-thrimethylbicyclo [2.2.1]hept-2-yl radical or a bicycle[3.2.1]oct-3-yl radical, and its salts.

13. The pharmaceutical according to claims 11 or 12, wherein g₄ is a halogen, w₄ is a C₁-C₆ alkyl group, R₁, R₃, R₄, R₅ are hydrogen.

14. Pharmaceutical composition according to any one of the preceding claims wherein the CB₂R receptor antagonist is a compound of formula I and wherein the further anti-protozoal active ingredients are selected from at least one of the group of chloroquine, chinine, and artemisinine.
